# EUROPÄISCHE PATENTSCHRIFT

(11) **EP 3 527 166 B1**
(45) Veröffentlichungstag und Bekanntmachung des Hinweises auf die Patenterteilung: **12.08.2020**
(21) Anmeldenummer: 19156820.3
(22) Anmeldetag: 13.02.2019
(51) Int. Cl.: A61C 13/08, A61C 13/00, A61K 6/80

(54) **DENTALE FORMKÖRPER MIT KONTINUIERLICHEM FARBVERLAUF**
DENTAL MOULDED BODY WITH CONTINUOUS COLOUR GRADIENT
CORPS DENTAIRE MOULÉ À DÉGRADÉ DE COULEUR CONTINU

(30) Priorität: 15.02.2018 DE 102018103415
(43) Veröffentlichungstag der Anmeldung: 21.08.2019
(73) Patentinhaber: VOCO GmbH, 27472 Cuxhaven (DE)
(72) Erfinder: Oldenburger, Daniel, 27476 Cuxhaven Niedersachsen (DE); Appelmann, Marie, 27476 Cuxhaven (DE); Plaumann, Manfred Thomas, 27472 Cuxhaven Niedersachsen (DE)

(56) Entgegenhaltungen:
- EP-A1- 2 995 434
- EP-A1- 3 173 048
- WO-A1-2013/156483
- WO-A1-2015/051095
- D Dietschi ET AL: "A new shading concept based on natural tooth color applied to direct composite restorations.", Quintessence Int. 2006 Feb;37(2):91-102., 1. Februar 2006 (2006-02-01), Seiten 91-102, XP55354199, Gefunden im Internet: URL:http://www.ncbi.nlm.nih.gov/pubmed/164 75370 [gefunden am 2017-03-13]

## Beschreibung

Die vorliegende Erfindung betrifft mehrfarbige dentale Fräsrohlinge auf Kunststoff- bzw. Kompositbasis zur Herstellung von Zahnrestaurationen in einem CAD/CAM Verfahren sowie ein Verfahren zur Herstellung dieser mehrfarbigen dentalen Fräsrohlinge.

Prothetische Versorgungen bilden eine Restauration bzw. einen Ersatz für Zähne und umfassen beispielsweise Kronen und Brücken, Teilkronen, Inlays, Onlays oder Verblendungen.

Der technische Fortschritt computergesteuerter Maschinen brachte es mit sich, dass Fräsmaschinen entwickelt wurden, die in der Lage sind, in kürzester Zeit und mit minimalem Aufwand prothetische Versorgungen in nicht gekannter Genauigkeit herzustellen. Vor diesem Hintergrund entwickelte sich die sogenannte "digitale Zahnmedizin". Sie ist heutzutage in Zahnmedizin, Zahntechnik sowie Medizintechnik und damit im Praxis- und Laboralltag von Zahnmedizinern von überragender Bedeutung.

Gefräst wurden anfangs ausschließlich keramische oder metallische Materialien, doch im Zuge der immer besser der natürlichen Zahnhartsubstanz angepassten dentalen Kompositmaterialien, wurde auch diese Substanzklasse für eine Verwendung als Fräsrohling interessant.

Anders als Kompositmaterialien, die durch gezielte Formulierung von Harzmatrix und Füllstoffzusammensetzung auf die mannigfachen Anforderungen eines Dentalmaterials in dem feindlichen Milieu der Mundhöhle eingestellt werden können, können keramische Materialien zu hohe Härtegrade aufweisen und neigen aufgrund ihrer inhärenten Sprödigkeit zur Bruchbildung. Metallische Materialien lassen sich ästhetisch schlecht einstellen und verursachen bei Patienten oft allergische Reaktionen.

Neben guten mechanischen Eigenschaften steigen zunehmend die Ansprüche an die ästhetischen Eigenschaften der prothetischen Versorgungen. So soll das natürliche Aussehen der Zähne möglichst genau nachempfunden werden.

Da natürliche Zähne nicht einfarbig sind, sondern einen komplexen Farbverlauf mit sich ändernder Farbe und Transluzenz aufweisen, sind Versorgungen aus einfarbigen Fräsrohlingen nicht in der Lage das natürliche Erscheinungsbild wiederzugeben.

Einfarbige Versorgungen lassen sich nachträglich mit Kompositmalfarben individualisieren oder individuell verblenden. Diese Verfahren sind aufwendig sowie zeit- und kostenintensiv.

Es wurden daher unterschiedliche mehrfarbige dentale Fräsrohlinge vorgeschlagen.

WO 2008/083358 A1 beschreibt dentale Fräsrohlinge, die unterschiedlich farbige innere und äußere Bereiche aufweisen.

DE 197 14 178 C2 beschreibt die Herstellung von mehrfarbigen, im Wesentlichen keramischen Formkörpern. Durch Verpressen einzelner, unterschiedlich gefärbter Schichten sollen die Farbübergänge zwischen den einzelnen Schichten weniger wahrnehmbar sein.

Auch DD 758 675 beschreibt die Herstellung künstlicher Zähne durch Pressen verschiedener Farbschichten. Dazu werden unterschiedlich gefärbte Kunststoffe nacheinander individuell verpresst.

EP 0 482 000 beschreibt mehrfarbige Kunststoff-Blöcke zur Herstellung von künstlichen Zähnen oder Kronen. Die Blöcke enthalten unterschiedlich gefärbte, diskrete Schichten.

EP 3 173 048 A1 beschreibt mehrfarbige Kunststoff-Blöcke zur Herstellung von Kronen und Brücken. Die Blöcke enthalten ebenfalls unterschiedlich gefärbte, diskrete Schichten.

WO 02/09612 A1 beschreibt mehrfarbige CAD/CAM-Blöcke aus Komposit, Kunststoff oder Keramik zur Herstellung von Kronen, Brücken, Inlays und Zahnersatz. Die Blöcke enthalten ebenfalls unterschiedlich gefärbte, diskrete Schichten, wobei die helleren, transluzenten Schichten die Schmelzbereiche und die dunkleren, opakeren Schichten die Dentinbereiche nachbilden sollen.

EP 1 900 341 B1 beschreibt mehrfarbige, im Wesentlichen keramische Formkörper mit aufeinander gelagerten Schichten. Durch einen gegenläufigen Farbverlauf von Haupt- und Zwischenschichten soll das menschliche Auge diese Schichten nicht einzeln wahrnehmen.

EP 2 829 251 A1 und US 9,649,179 B2 beschreiben mehrfarbige, dentale Fräsrohlinge aus Zirkonoxid. Die Fräsrohlinge bestehen aus unterschiedlich gefärbten Schichten.

EP 0 772 424 A1 beschreibt mehrfarbige, mehrschichtige künstliche Zähne, die durch Spritzguss hergestellt werden.

Alle diese bekannten, mehrfarbigen, dentalen Fräsrohlinge bzw. künstlichen Zähne, die aus unterschiedlich farbigen Schichten oder Bereichen bestehen, haben den Nachteil, dass sich durch die einzelnen, diskreten Schichten oder Bereiche kein natürlicher, kontinuierlicher Farbverlauf ergibt. Vielmehr werden Übergänge zwischen den einzelnen Schichten oder Bereichen wahrgenommen.

EP 1 859 758 A1 beschreibt die Herstellung von keramischen dentalen Blockkörpern mit kontinuierlichem Farbverlauf. Da hier die Herstellung über keramische Pulver erfolgt, lässt sich das Herstellungsverfahren nicht auf moderne kunststoffbasierte Fräsrohlinge übertragen.

WO 2015/051095 A1 beschreibt u.a. auch Fräsrohlinge auf Kunststoff- bzw. Kompositbasis, die aus mehreren Schichten mit unterschiedlichen Farben bestehen. Diese Fräsrohlinge weisen Farbsprünge zwischen den einzelnen schichten auf.

Aufgabe der vorliegenden Anmeldung war es daher kompositbasierte, dentale Fräsrohlinge mit einem kontinuierlichen Farbverlauf zur Verfügung zu stellen, die in der Lage sind den natürlichen Farbverlauf der Zähne nachzuempfinden und aus denen sich somit in CAD/CAM-Verfahren ästhetische Restaurationen fertigen lassen.

Gleichzeitig soll der kontinuierliche Farbverlauf nicht auf Kosten der mechanischen Eigenschaften eingestellt werden. Vielmehr sollen die Fräsrohlinge sowohl einen kontinuierlichen Farbverlauf und damit ein ästhetisches Erscheinungsbild als auch gute mechanische Eigenschaften aufweisen.

Erfindungsgemäß wird die Aufgabe gelöst durch einen Fräsrohling zur Herstellung einer indirekten dentalen Restauration aus Kunststoff oder aus einem kunststoffbasierten Komposit, dadurch gekennzeichnet, dass sich die Farbe kontinuierlich von einer Fläche zur gegenüberliegenden Fläche ändert, wobei sich der L*-Wert und der a* Wert und der b* Wert im L*a*b*-Farbraum kontinuierlich von einer Fläche zur gegenüberliegenden Fläche ändern und wobei die Differenz die Differenz ΔL* = |L*2 - L*1| größer 2 und die Differenz Δa* = |a*1 - a*2| größer 0.5 und die Differenz Δb* = |b*1 - b*2| größer 1 ist, wobei die L*a*b*-Werte an 2 mm dicken Scheiben gegen einen weißen Hintergrund gemessen werden.

Ein bevorzugter Fräsrohling zeichnet sich dadurch aus, dass sich der L*-Wert im L*a*b*-Farbraum von einem Wert L*1 an einer ersten Ecke und/oder Kante und/oder Fläche kontinuierlich auf einen Wert L*2 jeweils an der gegenüberliegenden Ecke und/oder Kante und/oder Fläche ändert, wobei L*2 > L*1 ist
und/oder
wobei sich der a*-Wert im L*a*b*-Farbraum von einem Wert a*1 an einer ersten Ecke und/oder Kante und/oder Fläche kontinuierlich auf einen Wert a*2 jeweils an der gegenüberliegenden Ecke und/oder Kante und/oder Fläche ändert, wobei a*2 < a*1 ist
und/oder
wobei sich der b*-Wert im L*a*b*-Farbraum von einem Wert b*1 an einer ersten Ecke und/oder Kante und/oder Fläche kontinuierlich auf einen Wert b*2 jeweils an der gegenüberliegenden Ecke und/oder Kante und/oder Fläche ändert, wobei b*2 < b*1 ist.

Ein bevorzugter Fräsrohling zeichnet sich dadurch aus, dass die Differenz ΔL* = |L*2 - L*11 größer 2 bevorzugt größer 4 und/oder
die Differenz Δa* = |a*1 - a*2| größer 0.5 bevorzugt größer 1 und/oder
die Differenz Δb* = |b*1 - b*2| größer 1 bevorzugt größer 2 ist.

Bei einem bevorzugten Fräsrohling liegen
L*1 im Bereich von 50 bis 70, bevorzugt von 50 bis 68,
L*2 im Bereich von 65 bis 85, bevorzugt von 68 bis 85,
a*1 im Bereich von 3 bis 7, bevorzugt von 3.5 bis 7,
a*2 im Bereich von -2 bis 4, bevorzugt von -2 bis 3.5,
b*1 im Bereich von 12 bis 22, bevorzugt von 14 bis 22, und
b*2 im Bereich von 5 bis 15, bevorzugt von 5 bis 14.

CAD/CAM-Rohlinge werden in Form von Würfeln, Quadern, Zylindern oder Scheiben angeboten.

Ein erfindungsgemäßer Fräsrohling weist x-, y- und z-Dimensionen von mindestens 5 mm, bevorzugt mindestens 10 mm besonders bevorzugt mindestens 14 mm auf.

Bevorzugt sind die Abmessungen des Fräsrohlings so gewählt, dass aus ihm
- ein Würfel mit einer Kantenlänge von 10 mm, vorzugsweise 14 mm,
   und/oder
- ein Quader mit einer quadratischen Grundfläche mit einer Kantenlänge von 10 mm, vorzugsweise 14 mm, und einer Höhe von 20 mm
   fräsbar ist.

Damit lassen sich aus einem solchen Fräsrohling dentale Formkörper, insbesondere Kronen, Teilkronen, Brücken, Inlays, Onlays, Veneers (Verblendungen), teil- oder vollanatomische künstliche Zähne oder Abutments herstellen.

Unter "kontinuierlicher Farbverlauf" wird im Rahmen dieser Anmeldung verstanden, dass sich die Farbwerte (L*, a*, b* im CIE 1976 L*a*b*-Farbraum - DIN EN ISO 11664-4:2011 Farbmetrik - Teil 4: CIE 1976 L*a*b* Farbenraum) innerhalb des Fräsrohlings (mit den Dimensionen x, y, z) nicht sprunghaft ändern. Mathematisch betrachtet sind damit die Funktionen L*(x,y,z), a*(x,y,z) und b*(x,y,z) stetig, d.h. sie weisen keine Sprünge auf. Im Gegensatz dazu ändern sich die Farbwerte bei einem Fräsrohling aus dem Stand der Technik mit mehreren diskreten, unterschiedlich farbigen Schichten sprunghaft von einer Schicht zur nächsten.

Unter "diametral gegenüberliegenden Ecken" wird im Rahmen dieser Anmeldung verstanden, dass bei einem Würfel oder Quader zwischen diametral gegenüberliegenden Ecken eine Raumdiagonale liegt. Die diametral gegenüberliegende Ecke ist also die Ecke, die von der ersten Ecke am weitesten entfernt liegende Ecke.

Unter "gegenüberliegenden Kanten" wird im Rahmen dieser Anmeldung verstanden, dass bei einem Würfel oder Quader zwischen gegenüberliegenden Kanten eine Diagonalfläche liegt. Die gegenüberliegende Kante ist also die Kante, die von der ersten Kante am weitesten entfernt liegende Kante.

Unter "gegenüberliegenden Fläche" wird im Rahmen dieser Anmeldung verstanden, dass zwischen gegenüberliegenden Flächen der Raum des Fräsrohlings liegt. Bei einem Würfel oder Quader sind dies parallele Flächen, die nicht aneinander grenzen. Bei einer Scheibe (Zylinder) sind dies die Ober- und die Unterseite der Scheibe.
Abbildung 1: Darstellung der Dosiergeschwindigkeit für die Co-Extrusion von zwei Pasten
Abbildung 2: Farbverlauf (L*-Wert) für Beispiel 2
Abbildung 3: Farbverlauf (a*-Wert) für Beispiel 2
Abbildung 4: Farbverlauf (b*-Wert) für Beispiel 2
Abbildung 5: Farbverlauf (L*-Wert) für Beispiel 3
Abbildung 6: Farbverlauf (a*-Wert) für Beispiel 3
Abbildung 7: Farbverlauf (b*-Wert) für Beispiel 3
Kompositbasierte, dentale Fräsrohlinge lassen sich herstellen, indem die Einzelkomponenten vermischt und zu einer homogenen Paste geknetet werden. Diese härtbare Kompositpaste wird in Formen abgefüllt und dort ausgehärtet.

Das Befüllen erfolgt dabei vom Boden der Form nach oben. Je nachdem, ob sich dabei eine Ecke, Kante oder Fläche unten befindet wird beim Befüllen also die Form von der unten liegenden Ecke zur gegenüberliegenden oberen Ecke oder von der unten liegenden Kante zur gegenüberliegenden oberen Kante oder von der unten liegenden Fläche zur gegenüberliegenden oberen Fläche befüllt.

Ein bevorzugtes Verfahren zur Herstellung eines Fräsrohlings für die Herstellung einer indirekten dentalen Restauration aus Kunststoff oder aus einem kunststoffbasierten Komposit, umfasst die folgenden Schritte
- Herstellen oder Bereitstellen einer ersten radikalisch härtbaren Zusammensetzung mit einer ersten Farbe,
- Herstellen oder Bereitstellen einer zweiten radikalisch härtbaren Zusammensetzung mit einer zweiten Farbe,
- Befüllen einer Form unter Mischen der ersten und der zweiten radikalisch härtbaren Zusammensetzungen und
- Aushärten der Mischung der ersten und der zweiten Zusammensetzung,
wobei das Mischungsverhältnis der ersten und der zweiten radikalisch härtbaren Zusammensetzungen beim Befüllen der Form von einer Fläche zur gegenüberliegenden Fläche kontinuierlich variiert wird.

Bevorzugt werden nach diesem Verfahren oben beschriebene mehrfarbige Fräsrohlinge hergestellt.

Vorteilhaft ist ein Verfahren, wobei das Mischen und Befüllen mit einer Förder- oder Pressvorrichtung, bevorzugt mit einer Extrusionsvorrichtung erfolgt.

Vorteilhaft wird mit einem Extrusionsdruck im Bereich von 20 bis 200 bar und/oder einer Extrusionsgeschwindigkeit im Bereich von 0.1 bis 100 mm/min, bevorzugt von 1 bis 30 mm/min, befüllt.

Vorteilhaft wird unter Anlegen eines Vakuums, bevorzugt im Bereich von -0.5 bis - 1.0 bar, befüllt. Unter einem Vakuum von -0.5 bar wird ein Unterdruck von 0.5 bar verstanden, so dass in diesem Fall der Restdruck 0.5 bar beträgt. Dementsprechend beträgt bei einem Vakuum von -0.85 bar der Unterdruck 0.85 bar und der Restdruck 0.15 bar.

Es wurde gefunden, dass sich mehrfarbige, dentale Fräsrohlinge herstellen lassen, indem zwei oder mehr unterschiedlich gefärbte Kompositpasten während des Abfüllens gemischt und dabei die Geschwindigkeit der Dosierung der einzelnen Pasten relativ zu einander geändert wird. Zum Beispiel kann eine erste Paste mit einer konstanten Geschwindigkeit dosiert werden und eine zweite Paste kann beim Abfüllvorgang mit stetig steigender Dosiergeschwindigkeit zugemischt werden. Es ist aber auch möglich eine erste Paste mit einer konstanten Geschwindigkeit zu dosieren und die zweite Paste mit abnehmender Dosiergeschwindigkeit zuzumischen. Füllt man nun abwechselnd eine Form mit steigender Dosiergeschwindigkeit der zweiten Paste und eine Form mit abnehmender Dosiergeschwindigkeit der zweiten Paste, ist es möglich abwechselnd Fräsrohlinge mit Farbverlauf in die eine und in die andere Richtung herzustellen. Dies hat den Vorteil, dass zwischen den einzelnen Füllvorgängen kein Material aus dem Mischer verworfen werden muss oder der Mischer gewechselt werden muss, damit man immer wieder mit der gleichen Farbe anfangen kann zu befüllen. Da der Farbverlauf kontinuierlich von einer Seite zur anderen ist und die Blöcke spiegelsymmetrisch sind, sind beide Blöcke nachher gleich.

Es ist aber auch möglich gleichzeitig die Dosiergeschwindigkeit der ersten Paste zu erhöhen und die Dosiergeschwindigkeit der zweiten Paste zu senken, so dass die Dosiergeschwindigkeit der Mischung insgesamt konstant bleibt. Dieses Vorgehen ist bevorzug, wenn sich bedingt durch die Abfüllanlage Grenzen für den Druckbereich, die einzelnen Dosiergeschwindigkeiten oder die Abfüllgeschwindigkeit ergeben. Auch hier können durch eine Umkehrung der einzelnen Dosiergeschwindigkeiten abwechselnd Blöcke mit Farbverlauf in die eine und in die andere Richtung hergestellt werden, so dass zwischenzeitlich kein Material aus dem Mischer verworfen oder der Mischer gewechselt werden muss.

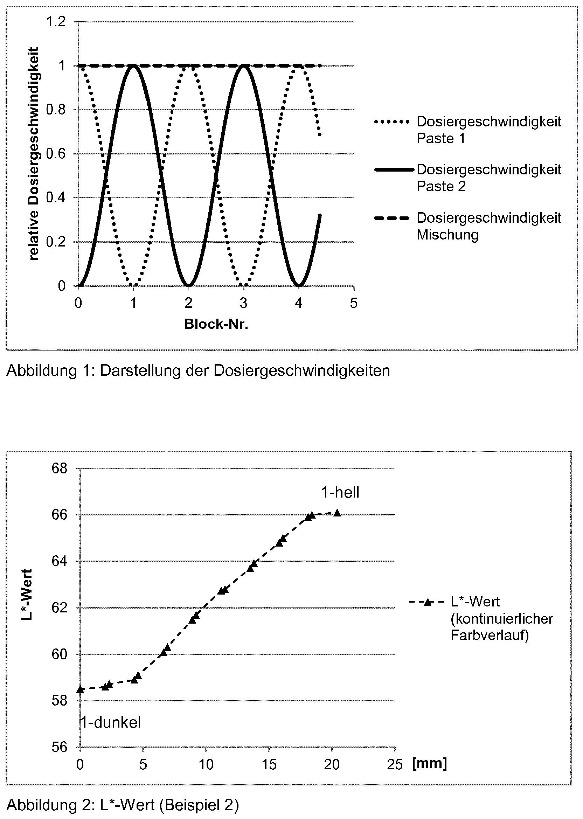

In Abbildung 1 ist eine Dosierung dargestellt, bei der die Dosiergeschwindigkeit der ersten Paste (gepunktete Linie) bei der Befüllung der ersten Form kontinuierlich sinkt während die Dosiergeschwindigkeit der zweiten Paste (durchgehende Linie) beim Befüllen der ersten Form kontinuierlich steigt. Die Dosiergeschwindigkeit der Mischung (gestrichelte Linie) bleibt dabei konstant. Der erste Block (Bereich zwischen 0 und 1 auf der x-Achse) hat damit einen Farbverlauf, bei dem die Farbe sich kontinuierlich von der Farbe der ersten Paste zur Farbe der zweiten Paste ändert. Bei der Befüllung der zweiten Form wird umgekehrt die Dosiergeschwindigkeit der ersten Paste (gepunktete Linie) kontinuierlich erhöht und die Dosiergeschwindigkeit der zweiten Paste (durchgehende Linie) kontinuierlich gesenkt. Der zweite Block (Bereich zwischen 1 und 2 auf der x-Achse) hat damit einen entsprechend umgekehrten Farbverlauf von der Farbe der zweiten Paste zur Farbe der ersten Paste. Vorteilhaft ändert sich die Dosiergeschwindigkeit der einzelnen Pasten sinusförmig. Damit wird die Steuerung der Dosierung vereinfacht und die Gefahr von Farbsprüngen beim Übergang von einem Block zum nächsten reduziert. Zudem können Haltezeiten am Anfang und am Ende der Befüllung einer Form vorgesehen werden, bei denen die Dosiergeschwindigkeiten der Pasten konstant bleiben.

Prinzipiell ist dieses Verfahren auch wunderbar geeignet, um direkt künstliche Zähne mit kontinuierlichem Farbverlauf herzustellen. Dazu wird eine Form gewählt, die der gewünschten teil- oder vollanatomischen Zahnform entspricht. Diese wird dann entsprechend mit zwei unterschiedlich gefärbten Pasten befüllt wobei sich die Dosiergeschwingkeit der beiden Pasten relativ zu einander kontinuierlich verändert.

Ein solches Verfahren zur Herstellung eines künstlichen Zahns aus Kunststoff oder aus einem kunststoffbasierten Komposit, umfasst somit die folgenden Schritte
- Herstellen oder Bereitstellen einer ersten radikalisch härtbaren Zusammensetzung mit einer ersten Farbe,
- Herstellen oder Bereitstellen einer zweiten radikalisch härtbaren Zusammensetzung mit einer zweiten Farbe,
- Befüllen einer Form unter Mischen der ersten und der zweiten radikalisch härtbaren Zusammensetzungen und
- Aushärten der Mischung der ersten und der zweiten Zusammensetzung,
wobei das Mischungsverhältnis der ersten und der zweiten radikalisch härtbaren Zusammensetzungen beim Befüllen kontinuierlich variiert wird.

Ein nach diesem Verfahren hergestellter künstlicher Zahn weist einen kontinuierlichen Farbverlauf von basal nach inzisal oder okklusal auf und entspricht damit dem natürlichen Erscheinungsbild eines Zahns.

Um möglichst gute mechanische Eigenschaften zu erzielen, ist ein möglichst hoher Füllstoffgehalt nötig. Gleichzeitig sorgt ein hoher Füllstoffgehalt aber für eine höhere Viskosität. Diese stellt eine Herausforderung für das Mischen und Abfüllen solcher Kompositpasten dar.

Um solche hochviskosen Kompositpasten homogen mischen und abfüllen zu können, kann z.B. mit Kolbenstrangpressen, Schneckenextrudern oder Kolbenextrudern gearbeitet werden. Dabei werden die einzelnen Kompositpasten bevor sie aufeinander treffen in den jeweiligen Druckbehältern der Abfüllmaschine (Kolbenstrangpresse, Doppelkolbenstrangpresse, Schneckenextruder, Kolbenextruder) unter Vakuum (von -0.5 mbar bis -1.0 mbar) kompaktiert, um so Lufteinschlüsse zu minimieren. Die Materialien werden anschließend durch ein geeignetes Mischelement extrudiert. Es ist dabei sowohl möglich einzelne Formen nacheinander zu befüllen und anschließend das Material in den Formen auszuhärten als auch das extrudierte Material direkt und kontinuierlich auszuhärten.

Das Aushärten des Komposits kann anschließend durch Foto- und/oder thermische Polymerisation erfolgen. Während des Härtens kann Druck angewendet werden, um so noch mögliche Materialdiskontinuitäten zu verringern oder vollständig zu entfernen.

Nachfolgend wird ein bevorzugter Fräsrohling aus kunststoffbasiertem Komposit beschrieben. Die Angaben zur Zusammensetzung treffen analog auf bevorzugte künstliche Zähne zu.

Bevorzugt ist ein erfindungsgemäßer Fräsrohling aus kunststoffbasiertem Komposit, umfassend
a) einen anorganischen Füllstoff in einer Menge von wenigstens 70 Gew.-%, vorzugsweise wenigstens 80 Gew.-%, bezogen auf die Gesamtmasse des Fräsrohlings
   und
b) eine Harzmatrix (Kunststoffmatrix).

Vorzugsweise ist ein bevorzugter erfindungsgemäßer Fräsrohling aus kunststoffbasiertem Komposit, der einen anorganischen Füllstoff in der vorstehend angegebenen Menge sowie eine Harzmatrix umfasst, so ausgestaltet, dass der anorganische Füllstoff a) umfasst:
a1) eine Glaszusammensetzung und
a2) nicht aggregierte und nicht agglomerierte Kieselsäure mit einer mittleren Teilchengröße von nicht mehr als 80 nm.

Besonders vorteilhaft ist insoweit ein erfindungsgemäßer Fräsrohling, wobei die Glaszusammensetzung a1)
eine erste Glaszusammensetzung a1a) mit einem D50-Wert im Bereich von 0,4 bis 1,0 µm, vorzugsweise im Bereich von 0,5 bis 0,9 µm,
und
eine zweite Glaszusammensetzung a1b) mit einem D50-Wert im Bereich von 1,2 bis 5,0 µm, vorzugsweise im Bereich von 1,5 bis 4,0 µm,
umfasst,
wobei das Massenverhältnis von a1a) zu a1b) zwischen 1 : 1,5 und 1 : 8, vorzugsweise zwischen 1 : 2 und 1 : 5 liegt,
wobei das Massenverhältnis von a2) zur Summe von a1a) und a1b) zwischen 1 : 3 und 1 : 6 liegt,
wobei das Verhältnis des D50-Werts der ersten Glaszusammensetzung a1a) zum D50-Wert der zweiten Glaszusammensetzung a1b) im Bereich von 1 : 1,5 bis 1 : 10, vorzugsweise 1 : 2 bis 1 : 5, liegt,
und wobei der D75-Wert der ersten Glaszusammensetzung a1a) kleiner ist als der D25-Wert der zweiten Glaszusammensetzung a1b).

In erfindungsgemäßen Fräsrohlingen aus kunststoffbasiertem Komposit, die ein anorganischen Füllstoff a) und eine Harzmatrix b) umfassen, kann die Harzmatrix bis zu 30 Gew.-% ausmachen, bezogen auf die Gesamtmasse des Fräsrohlings. Es sind jedoch häufig neben den Bestandteilen anorganischer Füllstoff a) und Harzmatrix b) auch ein oder mehrere weitere Bestandteile vorgesehen, die weder dem anorganischen Füllstoff noch der Harzmatrix zugerechnet werden, beispielsweise Reste von Initiatoren für die Härtung sowie sonstige Additive einschließlich etwaiger organischer Füllstoffe.

Ein bevorzugter erfindungsgemäßer Fräsrohling besteht aus einem kunststoffbasierten Komposit und umfasst
a) einen anorganischen Füllstoff in einer Menge von wenigstens 70 Gew.-%, vorzugsweise wenigstens 80 Gew.-%, bezogen auf die Gesamtmasse des Fräsrohlings.
   und
b) eine Harzmatrix,
wobei der anorganische Füllstoff a) umfasst:
a1) eine Glaszusammensetzung und
a2) nicht aggregierte und nicht agglomerierte Kieselsäure mit einer mittleren Teilchengröße von nicht mehr als 80 nm,
wobei die Glaszusammensetzung a1)
eine erste Glaszusammensetzung a1a) mit einem D50-Wert im Bereich von 0,4 bis 1,0 µm, vorzugsweise im Bereich von 0,5 bis 0,9 µm,
und
eine zweite Glaszusammensetzung a1b) mit einem D50-Wert im Bereich von 1,2 bis 5,0 µm, vorzugsweise im Bereich von 1,5 bis 4,0 µm,
umfasst,
wobei das Massenverhältnis von a1a) zu a1b) zwischen 1 : 1,5 und 1 : 8, vorzugsweise zwischen 1 : 2 und 1 : 5 liegt,
wobei das Massenverhältnis von a2) zur Summe von a1a) und a1b) zwischen 1 : 3 und 1 : 6 liegt,
wobei das Verhältnis des D50-Werts der ersten Glaszusammensetzung a1a) zum D50-Wert der zweiten Glaszusammensetzung a1b) im Bereich von 1 : 1,5 bis 1 : 10, vorzugsweise 1 : 2 bis 1 : 5, liegt,
und wobei der D75-Wert der ersten Glaszusammensetzung a1a) kleiner ist als der D25-Wert der zweiten Glaszusammensetzung a1b).

Bevorzugt ist ein erfindungsgemäßer Fräsrohling, der a) einen anorganischen Füllstoff in einer Menge von wenigstens 70 Gew.-%, vorzugsweise wenigstens 80 Gew.-%, bezogen auf die Gesamtmasse des Fräsrohlings und b) eine Harzmatrix umfasst, wobei die Harzmatrix b) ein Polymerisat von Monomeren ist, die difunktionelle (Meth)acrylate enthalten, wobei der Gewichtsanteil von ethoxyliertem Bisphenol-A-Dimethacrylat mit einem durchschnittlichen Ethoxylierungsgrad von 2 bis 4 Ethoxygruppen pro Molekül größer ist als 40 Gew.-% und kleiner ist als 50 Gew.-%, bezogen auf die Gesamtmasse der Monomere.

Ein solcher Fräsrohling wird unter Verwendung einer Monomerenmischung hergestellt, die difunktionelle (Meth)acrylate enthält, wobei der Gewichtsanteil von ethoxyliertem Bisphenol-A-Dimethacrylat mit einem durchschnittlichen Ethoxylierungsgrad von 2 bis 4 Ethoxygruppen pro Molekül in der Monomerenmischung größer ist als 40 Gew.-% und kleiner ist als 50 Gew.-%, bezogen auf die Gesamtmasse der Monomere. Nach Vermischen der Monomerenmischung, eines oder mehrerer Initiatoren für die Härtung sowie optional vorhandener Additive mit der erforderlichen Menge von anorganischem Füllstoff erfolgt die Härtung der Monomerenmischung durch Polymerisation zur Harzmatrix b) in üblicher Weise, z.B. durch Strahlenhärtung (photochemisch) und/oder durch chemische Härtung (Redoxreaktion) und/oder thermisch.

Nachfolgend werden bevorzugte Bestandteile erfindungsgemäßer Fräsrohlinge bzw. von aushärtbaren Mischungen angegeben, aus denen erfindungsgemäße Fräsrohlinge herstellbar sind. Analog sind diese Bestandteile bevorzugt geeignet, um daraus künstliche Zähne herzustellen.

### a) anorganische Füllstoffe:

Der erfindungsgemäße Fräsrohling umfasst anorganische Füllstoffe in einer Menge von wenigstens 70 Gew.-%, vorzugsweise wenigstens 80 Gew.-%, bezogen auf die Gesamtmasse des Fräsrohlings, und dementsprechend enthalten aushärtbare Mischungen zur Herstellung eines erfindungsgemäßen Fräsrohlings anorganische Füllstoffe in einer Menge von mindestens 70 Gew.-%, vorzugsweise von mindestens 80 Gew.-%, bezogen auf die Gesamtzusammensetzung der Mischung. Anorganische Füllstoffe werden vorzugsweise als Mischung diverser Füllstofffraktionen eingesetzt; zur Optimierung der Produkteigenschaften werden anorganische Füllstoffe in unterschiedlichen Korngrößen in die Rezepturen eingebracht, wobei sie vorzugsweise eine multimodale, ganz bevorzugt eine bimodale Verteilung aufweisen.

Abhängig von den Bedürfnissen des Einzelfalls werden anorganische Füllstoffe in Form von kompakten Gläsern und/oder in Form unterschiedlicher Kieselsäuren in verschiedenen Größen und Zuständen (monodispers, polydispers) als Bestandteile erfindungsgemäßer Fräsrohlinge und entsprechender VorMischungen präferiert.

Geeignete anorganische Bestandteile sind beispielsweise amorphe Materialien auf der Basis von Mischoxiden aus SiO₂, ZrO₂ und/oder TiO₂ sowie Füllstoffe wie Quarz-Glaskeramik oder Glaspulver, Bariumsilikatgläser, Bariumfluorsilikatgläser, Strontiumsilikatgläser, Strontiumborsilikat, Li/Al-Silikatgläser, Bariumgläser, Calciumsilikate, Natriumaluminiumsilikate, Fluoraluminiumsilikatgläser, Oxide von Aluminium oder Silicium, Zeolithe, Apatit, Zirkonsilikate, schwerlösliche Metallsalze wie Bariumsulfat oder Calciumfluorid sowie röntgenopake Füllstoffe wie Ytterbiumfluorid.

Vorzugsweise enthält ein erfindungsgemäßer Fräsrohling Barium-Aluminium-Borsilikatgläser als Bestandteil der Füllstoffkomponente a).

Zum besseren Einbau in die Harzmatrix (Kunststoffmatrix; Polymermatrix) können die genannten Materialien organisch oberflächenmodifiziert sein; dies ist in vielen Fällen bevorzugt. Beispielhaft genannt sei die Oberflächenbehandlung anorganischer Füllstoffe mit einem Silan. Als Haftvermittler eignet sich besonders das Methacryloxypropyltrimethoxysilan.

Vorzugsweise enthält ein erfindungsgemäßer Fräsrohling oberflächenbehandelte Barium-Aluminium-Borsilikatgläser, vorzugsweise silanisierte Barium-Aluminium-Borsilikatgläser und am meisten bevorzugt mit Methacryloxypropyltrimethoxysilan behandelte Barium-Aluminium-Borsilikatgläser.

In erfindungsgemäßen Fräsrohlingen werden vorzugsweise und in Abhängigkeit von den Erfordernissen des Einzelfalls unterschiedliche Kieselsäuren eingesetzt.

Vorzugsweise, wie vorstehend im Zusammenhang mit dem Bestandteil a2) ausgeführt, enthalten erfindungsgemäße Fräsrohlinge nanoskalige Kieselsäuren, das heißt Kieselsäurepartikel mit einer mittleren Teilchengröße von nicht mehr als 80 nm. Diese Kieselsäuren sind vorzugsweise nicht aggregiert und nicht agglomeriert. Die Herstellung der nanoskaligen Kieselsäuren erfolgt auf bekannte Weise, zum Beispiel durch Flammpyrolyse, Plasmaverfahren, Gasphasenkondensation, Kolloidtechniken, Präzipitationsverfahren, Sol-Gel Verfahren, etc.

Sofern die nanoskaligen Kieselsäuren in nicht agglomerierter und nicht aggregierter Form vorliegen, liegen sie vorzugsweise in monodisperser Form vor. Dies ist besonders bevorzugt. Um eine gute Einbindung der Nanopartikel (Partikel mit einer mittleren Teilchengröße von nicht mehr als 80 nm) in die Harzmatrix (Polymermatrix; Kunststoffmatrix) einer radikalisch härtbaren dentalen Zusammensetzung zur Herstellung eines erfindungsgemäßen Fräsrohlings zu ermöglichen, sind die Oberflächen der nanoskaligen Kieselsäuren vorzugsweise organisch oberflächenmodifiziert, das heißt ihre Oberflächen weisen organische Strukturelemente auf. Beispielhaft genannt sei erneut die Oberflächenbehandlung der Füllstoffe mit einem Silan. Als Haftvermittler eignet sich auch für die besagten Nanopartikel besonders das Methacryloxypropyltrimethoxysilan.

Ein erfindungsgemäßer Fräsrohling enthält besonders bevorzugt oberflächenbehandelte nanoskalige, nicht agglomerierte und nicht aggregierte Kieselsäureteilchen mit einer mittleren Partikelgröße von nicht mehr als 80 nm, vorzugsweise silanisierte nanoskalige, nicht agglomerierte und nicht aggregierte Teilchen mit einer mittleren Partikelgröße von nicht mehr als 80 nm und am meisten bevorzugt mit Methacryloxypropyltrimethoxysilan behandelte, nanoskalige, nicht agglomerierte und nicht aggregierte Kieselsäureteilchen mit einer mittleren Partikelgröße von nicht mehr als 80 nm.

Kommerziell erhältliche nanoskalige, nicht agglomerierte und nicht aggregierte Kieselsole, die bei Herstellung eines erfindungsgemäßen Fräsrohlings vorzugsweise eingesetzt werden können, sind beispielsweise unter der Bezeichnung "NALCO COLLOIDAL SILICAS" (Nalco Chemical Co.), "Ludox colloidal silica" (Grace) oder "Highlink OG (Clariant) im Handel.

Vorzugsweise umfasst der Füllstoffanteil eines erfindungsgemäßen Fräsrohlings eine Mischung von a2) nicht aggregierter und nicht agglomerierter Kieselsäure mit einer mittleren Teilchengröße von nicht mehr als 80 nm und einem zweiten Füllstoff in Form von Mikropartikeln mit einer mittleren Partikelgröße im Bereich von 0,4 µm bis 5 µm. Bei diesem zweiten Füllstoff handelt es sich vorzugsweise um die vorstehend als Komponente a1) definierte Glaszusammensetzung eines erfindungsgemäßen Fräsrohlings. Durch die Kombination von Nanopartikeln, das heißt nicht aggregierter und nicht agglomerierter Kieselsäure mit einer mittleren Teilchengröße von nicht mehr als 80 nm mit Mikropartikeln (vorzugsweise Mikropartikeln einer Glaszusammensetzung, vgl. a1) oben) wird eine besonders vollständige und gleichmäßige Volumenfüllung des erfindungsgemäßen Fräsrohlings erreicht.

Innerhalb eines entsprechenden erfindungsgemäßen Fräsrohlings bewirken die Mikropartikel eine weitgehende gleichmäßige Füllung des Volumens, wobei die verbleibenden Hohlräume zwischen den Mikropartikeln durch die oben beschriebenen Nanopartikel (Komponente a2)) zumindest teilweise gefüllt werden. Unter Mikropartikeln werden im Zusammenhang mit der vorliegenden Erfindung Partikel mit einer mittleren Partikelgröße von 400 nm bis 5 µm verstanden. Der Einsatz von Glaszusammensetzungen als Mikropartikel ist bevorzugt.

Sofern im anorganischen Füllstoff a) eines bevorzugten erfindungsgemäßen Fräsrohlings Mikropartikel enthalten sind (vorzugsweise Mikropartikel einer Glaszusammensetzung a1)) weisen diese Mikropartikel vorzugsweise eine bimodale Partikelgrößenverteilung auf. Mikropartikel mit einer bimodalen Partikelgrößenverteilung sind bevorzugt, da mit ihnen eine vollständigere Volumenfüllung erreichbar ist als bei Verwendung von Mikropartikeln mit monomodaler Partikelgrößenverteilung. Bei Vorliegen einer bimodalen Partikelgrößenverteilung bewirken die Partikel der Fraktionen mit der größeren Partikelgröße eine grobe Ausfüllung des Volumens, während die Partikel der Fraktion mit der kleineren Partikelgröße soweit möglich die Bereiche zwischen den Partikeln der Fraktionen mit der größeren Partikelgröße ausfüllen werden. Die dann noch verbleibenden Hohlräume werden wie oben beschrieben durch Nanopartikel gefüllt.

Bevorzugt ist die Verwendung einer Mischung zweier Mikropartikelfraktionen, wobei eine erste Mikropartikelfraktion einen D50-Wert im Bereich von 0,4 bis 1,0 µm, vorzugsweise im Bereich von 0,5 bis 0,9 µm besitzt. Vorzugsweise handelt es sich hierbei um eine erste Glaszusammensetzung a1a) (siehe für bevorzugte Ausgestaltungen oben). Die zweite Mikropartikelfraktion besitzt einen D50-Wert im Bereich von 1,2 µm bis 5,0 µm, vorzugsweise im Bereich von 1,5 µm bis 4,0 µm. Vorzugsweise handelt es sich hierbei um eine zweite Glaszusammensetzung a1b) wie oben definiert (für bevorzugte Ausgestaltungen siehe oben).

Vorzugsweise liegt das Verhältnis der Gesamtmasse einer solchen ersten Mikropartikelfraktion zur Gesamtmasse einer solchen zweiten Mikropartikelfraktion im Bereich von 1 : 1,5 bis 1 : 8, vorzugsweise im Bereich von 1 : 2 bis 1 : 5. Dies gilt insbesondere dann, wenn die erste Mikropartikelfraktion eine erste Glaszusammensetzung a1a) ist und die zweite Mikropartikelfraktion eine zweite Glaszusammensetzung a1b) ist.

### b) Harzmatrix (Kunststoffmatrix, Polymermatrix) sowie Monomere zur Herstellung einer solchen Harzmatrix:

Ein erfindungsgemäßer Fräsrohling zur Herstellung einer indirekten dentalen Restauration bestehend aus Kunststoff oder einem kunststoffbasierten Komposit. Zur Herstellung des gehärteten Kunststoffs bzw. der Harzmatrix (Kunststoffmatrix; Polymermatrix, welche den Kunststoffanteil im kunststoffbasierten Komposit ausmacht), werden radikalisch polymerisierbare Mononmere als Bestandteil einer radikalisch aushärtbaren Zusammensetzung eingesetzt, die daneben noch anorganischen Füllstoff der Komponente a) sowie gegebenenfalls weitere Komponenten enthält. Der Anteil des Polymerisats der radikalisch polymerisierbaren Monomere in einem erfindungsgemäßen Fräsrohling ist vorzugsweise nicht höher als 30 Gew.-%, denn bevorzugt ist ein anorganischer Füllstoff in einer Menge von wenigstens 70 Gew.-% anwesend (siehe oben). Für die radikalisch härtbare Zusammensetzung, in der die radikalisch polymerisierbaren Monomere neben Füllstoffen eingesetzt werden, gilt entsprechendes.

Die radikalisch polymerisierbaren Monomere sind vorzugsweise die in der Dentalchemie üblicherweise in Kompositmaterialien eingesetzten (Meth)acrylatmonomere. Ein entsprechendes Polymerisat umfasst dann ein entsprechendes Poly(meth)acrylat. Unter (Meth)acrylaten werden im Rahmen dieser Anmeldung sowohl Methacrylate als auch Acrylate verstanden.

In der Patentliteratur ist eine Vielzahl von Verbindungen genannt (beispielsweise im Dokument DE 39 41 629 A1), die allesamt Diester der Acryl- oder Methacrylsäure sind und zur Herstellung eines Kunststoffs beziehungsweise einer Harzmatrix eines kunststoffbasierten Komposits geeignet sind, wie es in einem erfindungsgemäßen Fräsrohling vorliegt.

In einer bevorzugten Ausführung eines erfindungsgemäßen Fräsrohlings umfasst dieser Fräsrohling eine Harzmatrix, welche durch Polymerisation von ein oder mehreren Monomeren erzeugt wird, die ausgewählt sind aus der Gruppe bestehend Ethylenglykoldimethacrylat (EGDMA), 1,6-Hexandioldimethacrylat (HDDMA), Triethylenglykoldimethacrylat (TEGDMA), 1,10-Decandioldimethacrylat (DEDMA), 1,12-Dodecandioldimethacrylat (DODMA), ethoxyliertes Bisphenol-A-dimethacrylat, ethoxyliertes Bisphenol-A-dimethacrylat, wobei das Bisphenol mit 2 bis 4 mol Ethylenoxid umgesetzt wird und das Zwischenprodukt dann mit 2 mol Methacrylsäure abgesättigt wird, Polyethylenglykoldimethacrylat (PEGDMA), 7,7,9-Trimethyl-4,13-dioxo-3,14-dioxa-5,12-diazahexadecan-1,16-dioxydimethacrylat (U DMA), Butandioldimethacrylat, Tetraethylenglykoldimethacrylat, Neopentylglykoldimethacrylat sowie Bisphenol-A-Glycidyl-Methacrylat (Bis-GMA).

Im Einzelnen bevorzugt sind die entsprechenden Dimethacrylate bzw. Diacrylate des Dihydroxymethyltricyclo[5.2.1.0^{2,6}]decans, wie sie in den Druckschriften DE 1816823, DE 2419887, DE 2406557, DE 2931926, DE 3522005, DE 3522006, DE 3703120, DE 102005021332, DE 102005053775, DE 102006060983, DE 69935794 und DE 102007034457 beschrieben sind.

### c) Initiatoren:

Bevorzugte erfindungsgemäße Fräsrohlinge sind durch Strahlenhärtung (photochemisch) und/oder durch chemische Härtung (Redoxreaktion) und/oder thermisch durch Aushärtung ein entsprechenden Zusammensetzung herstellbar, wobei die Zusammensetzung als Komponente a) ein anorganischen Füllstoff in einer Menge von wenigstens 70 Gew.-%, vorzugsweise wenigstens 80 Gew.-%, bezogen auf die Gesamtmasse des hergestellten Fräsrohlings und/oder der eingesetzten Zusammensetzungen enthält oder eine füllstofffreie Zusammensetzung zur Herstellung eines Fräsrohlings aus Kunststoff ist. Erfindungsgemäß bevorzugt zur Herstellung eines Fräsrohlings ist die thermische Härtung einer entsprechenden Zusammensetzung, wobei die thermische Härtung beispielsweise durch Peroxidzerfall herbeigeführt wird.

Beispiele für geeignete Photosensibilisatoren sind alpha-Diketone, Benzoinalkylether, Thioxanthone, Benzophenone, Acylphosphinoxide, Acylgermanium-Verbindungen, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1, oder in der DE 39 41 629 C2.

Beispiele für Beschleuniger, die zusammen mit den Sensibilisatoren eingesetzt werden, sind tertiäre Amine, sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 oder in der DE 39 41 629 C2.

Weitere geeignete Initiatoren sowie Initiatorkombinationen sind in der DE 601 16 142 beschrieben.

Geeignete Photoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Coinitiatoren, die Aushärtung einer radikalisch härtbaren dentalen Zusammensetzung bewirken können.

Das Absorptionsmaximum von Campherchinon (CQ) liegt bei ca. 470 nm und somit im Bereich des blauen Lichts. Campherchinon (CQ) zählt zu den Pl2-Initiatoren und wird regelmäßig zusammen mit einem Coinitiator eingesetzt.

Ein geeignetes Katalysatorsystem enthält die Kombination eines alpha-Diketons und eines aromatischen tertiären Amins, bevorzugt ist die Kombination von Campherchinon (CQ) und Ethyl-p-N,N-dimethylaminobenzoat (DABE).

Ebenfalls bevorzugt ist die weitere Kombination des Systems "alpha-Diketon/aromatisches tertiäres Amin" mit einem Phosphinoxid, insbesondere mit dem Phenyl-bis(2,4,6-trimethylbenzoyl)phosphinoxid und/oder dem 2,4,6-Trimethylbenzoyl-diphenylphosphinoxid. Bezüglich der Strukturen geeigneter Phosphinoxide wird auf die Druckschriften DE 38 01 511 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 047 902 A, EP 0 007 508, DE 600 29 481 T2, EP 0 980 682 B1, EP 0 948 955 B1, EP 1 236 459 B1 und EP 0 173 567 A2 verwiesen.

Die in diesen Druckschriften angegebenen Phosphinoxide eigenen sich besonders allein oder in Kombination mit dem System "alpha-Diketon/Amin" als Photopolymerisationsi nitiatorsystem.

Weitere geeignete Photoinitiatoren sind in J.-P. Fouassier, Photoinitiation, Photopolymer-ization and Photocuring, Hanser Publishers, Munich, Vienna, New York 1995 sowie in J.F. Rabek (Hrsg.), Radiation Curing in Polymer Science and Technology, Vol. II, Elsevier Applied Science, London, New York 1993 beschrieben.

Dem Fachmann sind diverse Initiatoren für eine chemische Aushärtung bekannt. Es sei insoweit exemplarisch auf die EP 1 720 506 verwiesen. Initiatoren zur chemischen Aushärtung werden auch in den oben bereits erwähnten Druckschriften DE 10 2006 019 092 sowie in der DE 39 41 629 beschrieben.

Bevorzugte Initiatoren zur chemischen Härtung sind Dibenzoylperoxid, Dilauroylperoxid insbesondere Dibenzoylperoxid in Kombination mit Aminen wie N,N-Dimethyl-p-toluidin, N,N-Dihydroxyethyl-p-toluidin sowie strukturverwandten Aminen.

Dualhärtende Systeme umfassen eine Kombination aus Photoinitiatoren und Initiatoren zur chemischen Aushärtung.

Neben den oxidativ wirksamen organischen Peroxidverbindungen können als Redoxsysteme auch Barbitursäuren bzw. Barbitursäurederivate sowie Malonylsulfamide verwendet werden.

Von den Barbitursäuresystemen sind die sogenannten "Bredereck-Systeme" von hoher Bedeutung. Beispiele geeigneter "Bredereck-Systeme" sowie Verweise auf die entsprechende Patentliteratur findet man in der EP 1 839 640 sowie in DE 1495520, WO 02/092021 oder in WO 02/092023.

Anstelle der Barbitursäuren können auch deren Salze verwendet werden. Beispiele hierzu finden sich in den folgenden Dokumenten: EP 1 872 767, EP 2 070506, EP 1 881 010, DE 10 2007 050 763, US 6,288,138, DE 11 2006001 049, US 7,214,726 sowie EP 2 070 935.

Geeignete Malonylsulfamide sind in der EP 0 059 451 beschrieben. Bevorzugte Verbindungen sind dabei 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Diocytyl-4-isobutylmalonylsulfamid.

Ferner können Schwefelverbindungen in der Oxidationsstufe +2 oder +4 wie Natriumbenzolsulfinat oder Natriumparatoluolsulfinat eingesetzt werden.

Zur Beschleunigung der Aushärtung kann die Polymerisation in Gegenwart von Schwermetallverbindungen wie Ce, Fe, Cu, Mn, Co, Sn oder Zn durchgeführt werden, wobei Kupferverbindungen besonders bevorzugt sind. Die Schwermetallverbindungen werden bevorzugt in Form löslicher organischer Verbindungen eingesetzt. Bevorzugte Kupferverbindungen sind dabei Kupferbenzoat, Kupferacetat, Kupferethylhexanoat, Kupferdi(methacrylat), Kupferacetylacetonat und Kupfernaphthenat.

Werden die Peroxide erwärmt, so zerfallen sie und bilden freie Radikale, die befähigt sind, die Polymerisation zu starten. Das am weitesten verbreitete System zur thermischen Polymerisation ist die Verwendung von Dibenzoylperoxid. Weitere thermische Initiatoren sind Ketonperoxide, Peroxyketale, Hydroperoxide, Dialkylperoxide, Diacylperoxide, Peroxyester und Peroxydicarbonate wie Dicumylperoxid, Chlorbenzoylperoxid, tert.-Butylperbenzoat, Dilauroylperoxid, Cumolhydroperoxid, 3,5,5-Trimethylhexansäure-tert.-butylperoxyester sowie Azoverbindungen wie 2,2'-Azobisisobutyronitril, 2,2'-Azobis-2,4-dimethylvaleronitril, 2,2'-Azobis-1-cyclohexancabonitril oder Dimethyl-2,2'-azobisisobutyrat. Auch Substanzen wie Natrium- oder Kaliumpersulfat zerfallen thermisch und sind in diesem Zusammenhang geeignete Verbindungen. Diese Substanzen können einzeln oder in Mischungen untereinander verwendet werden. Hierzu müssen die radikalisch härtbaren dentalen Zusammensetzungen lediglich auf die vom Hersteller angegebene Zerfallstemperatur der jeweiligen Peroxide erwärmt werden. Vorteilhafterweise werden die radikalisch härtbaren Zusammensetzungen auf eine Temperatur oberhalb der Zerfallstemperatur erwärmt und dort für einige Zeit belassen, damit das Polymerisat Zeit zur Relaxation hat. Der Fachmann findet die optimale Temperatur dadurch, dass er die Temperatur zur Härtung sukzessive bis zu dem Punkt erhöht, an dem das Polymerisat keine wesentlichen Verbesserungen an den an ihm gemessenen wichtigen Parameter wie Biegefestigkeit, E-Modul und Wasseraufnahme aufweist.

Vorzugsweise wird die thermische Härtung so ausgeführt, dass die radikalisch härtbare Zusammensetzung in eine Blockform überführt wird, wo sie bei Temperaturen von 80°C bis 150°C und bei einem Druck von 100 bis 300 bar ausgehärtet wird.

### d) Additive:

Ein erfindungsgemäßer Fräsrohling umfasst in manchen Fällen ein oder mehrere weitere(s) Additiv(e).

Diese Additive können verschiedene Funktionen haben. Übliche Additive für den Einsatz in dentalen Werkstoffen sind dem Fachmann bekannt, je nach gewünschter Funktion wird er das oder die geeigneten Additive auswählen. Beispielhaft sind im Folgenden typische Additive und ihre Funktionen beschrieben.

UV-Absorber, die beispielsweise durch ihre konjugierten Doppelbindungssysteme und aromatischen Ringe befähigt sind, UV Strahlung zu absorbieren, sind in manchen Fällen Bestandteil eines erfindungsgemäßen Fräsrohlings. Beispiele von UV-Absorbern sind 2-Hydroxy-4-methoxybenzophenon, Salicylsäurephenylester 3-(2'-Hydroxy-5'-methylphenyl)-benzotriazol und Diethyl-2,5-dihydroxyterephthalat. Die Polymerisate enthalten diese Additive, um ihre Farbstabilität zu gewährleisten.

Da indirekte dentale Restaurationen dazu vorgesehen sind, Zähne möglichst naturgetreu zu restaurieren, ist es erforderlich, die erfindungsgemäßen Fräsrohlinge in unterschiedlichen Farbtönen bereitzustellen. Zu diesem Zweck enthalten erfindungsgemäße Fräsrohlinge in der Regel anorganische Farbstoffe und/oder organische Pigmente, vorzugsweise in sehr geringen, jedoch für die genannten Zwecke ausreichenden Mengen. Typische anorganische Pigmente sind z.B. Eisenoxide und Titandioxid. Farbmittel werden in einer international offiziellen Liste geführt, die von der Society of Dyers and Colourists in Bradford, England herausgegeben wird. Nach dem Generic Name folgt der Colour Index Cl.

### Beispiele:

### Abkürzungen:

Bis-EMA2,6: Ethoxyliertes Bisphenol A-Dimethacrylat mit durchschnittlich 2,6 Ethylenoxid-einheiten
Bis-GMA: Bisphenol-A-Glycidyl-Methacrylat
TCDDMA: Bis(methacryloyloxymethyl)tricyclo[5.2.1.0^{2,6}]decan
UDMA: 7,7,9-Trimethyl-4,13-dioxo-3,14-dioxa-5,12-diazahexadecan-1,16-dioxydimethacrylat
HDDMA: 1,6-Hexandioldimethacrylat
Dentalglas 1: Barium-Aluminium-Borosilikat-Glas (D50 0,8 µm / D25 0,5 µm / D75 1,0 µm), silanisiert
Dentalglas 2: Barium-Aluminium-Borosilikat-Glas (D50 2,7 µm / D25 1,4 µm / D75 6,1 µm), silanisiert
Nano-SiO₂: nicht agglomerierte, nicht aggregierte Kieselsäure (D50 40 nm), silanisiert
BPO: Dibenzoylperoxid

Farbmessung: Für die Messung der Farbwerte wurden die Kompositblöcke mit einer Präzisionssäge (IsoMet 4000, Buehler, Trennscheibendicke 0.3 mm) parallel zur Grundfläche in 2 mm dicke Scheiben gesägt. Mit einem Farbmessgerät ColorFlex EZ (CLFX EZ 45/0 LAV, HunterLab - Illuminant D65, 10° Observer ASTM E308) wurden jeweils für die Vorder- und Rückseite jeder Scheibe die CIE 1976 L*a*b*-Farbwerte gegen einen weißen Hintergrund (X:79.76, Y:84.89, Z:91.85 - Illuminant D65, 10° Observer ASTM E308) bestimmt. Für die mehrfarbigen Kompositblöcke ergibt sich damit ein Farbverlauf über den gesamten Block. Für die einfarbigen Vergleichsblöcke wurde nur eine 2 mm dicke Scheibe aus der Mitte des Blockes herausgesägt und an dieser die CIE 1976 L*a*b*-Farbwerte gegen einen weißen Hintergrund bestimmt.

Biaxiale Biegefestigkeit (BBF): Die Biaxiale Biegefestigkeit wurde analog DIN EN ISO 6872:2009 (7.3.3) bestimmt. Dazu wurden aus den Kompositblöcken zunächst in einer 5-Achs Fräsmaschine (250i, imes-icore GmbH) Zylinder mit einem Durchmesser von 14 mm geschliffen. Aus diesen Zylindern wurden dann mit einer Präzisionssäge (IsoMet 4000, Buehler) Scheiben mit einer Dicke von 1,2 mm hergestellt, entgratet, geschliffen und poliert. Die Proben wurden mit einer Traversengeschwindigkeit von 1 mm/min bis zum Bruch belastet und die biaxiale Biegefestigkeit entsprechend der unter 7.3.3.4 angegebenen Formel berechnet. Als Poisson'sche Querkontraktionszahl wurde ein Wert von 0,25 eingesetzt.

3-Punkt-Biegefestigkeit (3PBF): Die Biegefestigkeit wurde analog DIN EN ISO 6872:2009 (7.3.2) bei einer Stützweite von 12 mm und einem Auflagerollendurchmesser von 2 mm bestimmt. Dazu wurden aus den Kompositblöcken Probekörper mit einer Breite von 4 mm, einer Dicke von 1,2 mm und einer Länge von 18 mm mit einer Präzisionssäge (IsoMet 4000, Buehler) hergestellt, entgratet, geschliffen und poliert. Die Proben wurden mit einer Traversengeschwindigkeit von 1 mm/min bis zum Bruch belastet und die 3-Punkt-Biegefestigkeit entsprechend der unter 7.3.2.4.1 angegebenen Formel berechnet.

Elastizitätsmodul (E-Modul): Der Elastizitätsmodul wurde analog der Berechnung in ADA Specification No. 27 - 1993 (7.8.4.2) als Steigung der Spannung-Dehnungs-Kurve der 3-Punkt-Biegefestigkeitsmessung (siehe oben) im linearelastischen Bereich ermittelt.

Partikelgrößenbestimmung: Die Partikelgrößenbestimmung der Nanopartikel "Nano-SiO₂" erfolgte mittels Dynamic light scattering (DLS) mit einem Gerät des Typs Zetasizer Nano ZS (Malvern) bei 0,5 Gew.-% in 2-Butanon (Volumengewichtung). Die Partikelgrößenbestimmung der Mikropartikel (Dentalglas) erfolgte mittels Laserbeugung unter Verwendung eines Geräts des Typs Beckmann Coulter LS 13320.

### Beispiel 1 (Herstellung der ungefärbten Kompositpasten):

Für die Herstellung der ungefärbten Kompositpasten wurden die einzelnen Komponenten entsprechend der in Tabellen 1 angegebenen Anteile eingewogen und für 30 Minuten bei 50 U/min an einem Laborkneter (PC Laborsystem, Magden CH) homogenisiert. Zur Einfärbung wurden den ungefärbten Pasten kleine Mengen weißer, gelber, roter, brauner und schwarzer Farbpigmente zugesetzt. Dem Fachmann ist aus der Einfärbung von direkten Füllungsmaterialien auf Kompositbasis bekannt, welche Farbpigmente und in welchem Anteil er zu wählen hat, um eine Farbe zu erhalten, die die gewünschten Farbwerte aufweist und z.B. einer VITA-Farbe entspricht. Die gefärbten Pasten wurden am Laborkneter erneut für 30 Minuten bei 50 U/min homogenisiert und anschließend für 15 Minuten bei 50 U/min und -0,85 bar entlüftet.

**Tabelle 1 (Gewichtsanteile):**

| ungefärbte Kompositpaste | | | 1 | 2 |
|---|---|---|---|---|
| Füllstoff (a) | (a1a) | Dentalglas 1 | 11.00 | 12.00 |
| | (a1b) | Dentalglas 2 | 51.00 | 56.00 |
| | (a2) | Nano-SiO₂ (40 nm) | 18.00 | 12.00 |
| | Gesamt-(a) | | 80.00 | 80.00 |
| Monomere (b) | (b1a) | Bis-EMA2,6 | 8.00 | 9.00 |
| | (b2) | Bis-GMA | 3.50 | |
| | | TCDDMA | 3.50 | 5.00 |
| | | UDMA | 3.50 | 5.00 |
| | | HDDMA | 1.20 | 0.70 |
| | Gesamt-(b) | | 19.70 | 19.70 |
| Initiatoren (c) | | BPO | 0.30 | 0.30 |
| Gesamt | | | 100.00 | 100.00 |

### Beispiel 2:

Die ungefärbte Kompositpaste 1 wurde in zwei Versuchen unterschiedlich eingefärbt, so dass sich die beiden Farben deutlich voneinander unterschieden (Paste 1-hell und Paste 1-dunkel).

Die Kompositpasten 1-hell und 1-dunkel wurden jeweils durch Extrusion bei einem Druck von 100 bar und einem Vakuum von -0.85 bar in Formen (Grundfläche: 15 mm x 15 mm, Höhe 21 mm) gefüllt.

Für die Herstellung der erfindungsgemäßen Blöcke wurden die Kompositpasten 1-hell und 1-dunkel durch Co-Extrusion bei einem Vakuum von -0.85 bar in Formen (Grundfläche: 15 mm x 15 mm, Höhe 21 mm) gefüllt. Zunächst wurden Paste 1-dunkel mit 20 mm/min und Paste 1-hell mit 0 mm/min für neun Sekunden durch einen statischen Mischer dosiert. Anschließend wurde über einen Zeitraum von 45 Sekunden die Dosiergeschwindigkeit der Paste 1-hell kontinuierlich von 0 auf 20 mm/min erhöht während gleichzeitig die Dosiergeschwindigkeit der Paste 1-dunkel kontinuierlich von 20 auf 0 mm/min gesenkt wurde. Zum Schluss wurde noch für neun Sekunden die Paste 1-hell mit 20 mm/min dosiert (Paste 1-dunkel 0 mm/min).

Die Aushärtung erfolgte für alle Blöcke isostatisch bei 250 bar und folgendem Temperaturprogramm (20 °C - 2 °C/min - 120 °C (30 min) - 5 °C/min - 20 °C).

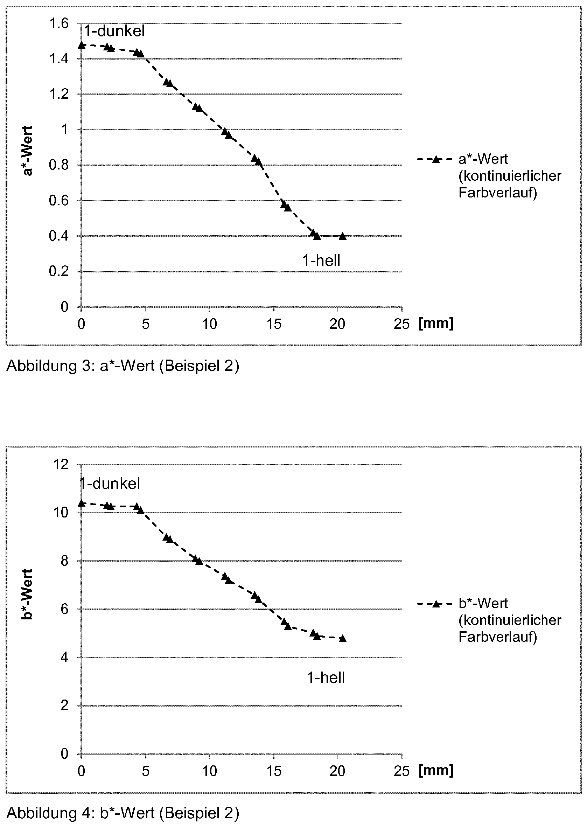

Die zwei einfarbigen Vergleichsblöcke weisen die in Tabelle 2 angegebenen L*a*b*-Farbwerte auf. Für den erfindungsgemäßen Block mit kontinuierlichem Farbverlauf sind die L*a*b*-Farbwerte in den Abbildungen 2 bis 4 dargestellt.

**Tabelle 2 (Farbwerte einfarbiger Blöcke aus den Pasten 1-hell und 1-dunkel):**

| | 1-hell | 1-dunkel |
|---|---|---|
| L*-Wert | 66.1 | 58.5 |
| a*-Wert | 0.4 | 1.5 |
| b*-Wert | 4.8 | 10.4 |

**Tabelle 3 (Biegefestigkeiten):**

| | 1-hell | 1-dunkel | Block mit kontinuierlichem Farbverlauf |
|---|---|---|---|
| BBF | 207 MPa | 201 MPa | 206 MPa |
| 3PBF | 181 MPa | 179 MPa | 181 MPa |
| E-Modul | 13.5 GPa | 13.4 GPa | 13.5 GPa |

### Beispiel 3:

Die ungefärbte Kompositpaste 2 wurde in drei unterschiedlichen Versuchen so eingefärbt, dass sie etwa den VITA-Farben A1 (Paste 2-A1), A2 (Paste 2-A2) und A3 (Paste 2-A3) entsprach.

Die Kompositpasten 2-A1, 2-A2 und 2-A3 wurden jeweils durch Extrusion bei einem Druck von 100 bar und einem Vakuum von -0.85 bar in Formen (Grundfläche: 15 mm x 15 mm, Höhe 21 mm) gefüllt.

Zusätzlich wurde eine Form (Grundfläche 15 mm x 15 mm, Höhe 21 mm) mit drei jeweils 7 mm dicken aufeinander folgenden Schichten der einzelnen Pasten 2-A1, 2-A2 und 2-A3 gefüllt.

Für die Herstellung der erfindungsgemäßen Blöcke wurden die Kompositpasten 2-A1 und 2-A3 durch Co-Extrusion bei einem Vakuum von -0.85 bar in Formen (Grundfläche: 15 mm x 15 mm, Höhe 21 mm) gefüllt. Zunächst wurden Paste 2-A1 mit 0 mm/min und Paste 2-A3 mit 10 mm/min für neun Sekunden durch einen statischen Mischer dosiert. Anschließend wurde über einen Zeitraum von 108 Sekunden die Dosiergeschwindigkeit der Paste 2-A1 von kontinuierlich 0 auf 10 mm/min erhöht während gleichzeitig die Dosiergeschwindigkeit der Paste 2-A3 kontinuierlich von 10 auf 0 mm/min gesenkt wurde. Zum Schluss wurde noch für neun Sekunden die Paste 2-A1 mit 10 mm/min dosiert (Paste 2-A3 0 mm/min).

Die Aushärtung erfolgte für alle Blöcke isostatisch bei 250 bar und folgendem Temperaturprogramm (20 °C - 2 °C/min - 120 °C (30 min) - 5 °C/min - 20 °C).

Die drei einfarbigen Vergleichsblöcke weisen die folgenden L*a*b*-Farbwerte auf.

**Tabelle 4 (Farbwerte einfarbiger Blöcke aus den Pasten 2-A1, 2-A2 und 2-A3):**

| | 2-A1 | 2-A2 | 2-A3 |
|---|---|---|---|
| L*-Wert | 73.0 | 69.0 | 66.6 |
| a*-Wert | 0.0 | 2.5 | 3.5 |
| b*-Wert | 9.5 | 13.5 | 17.0 |

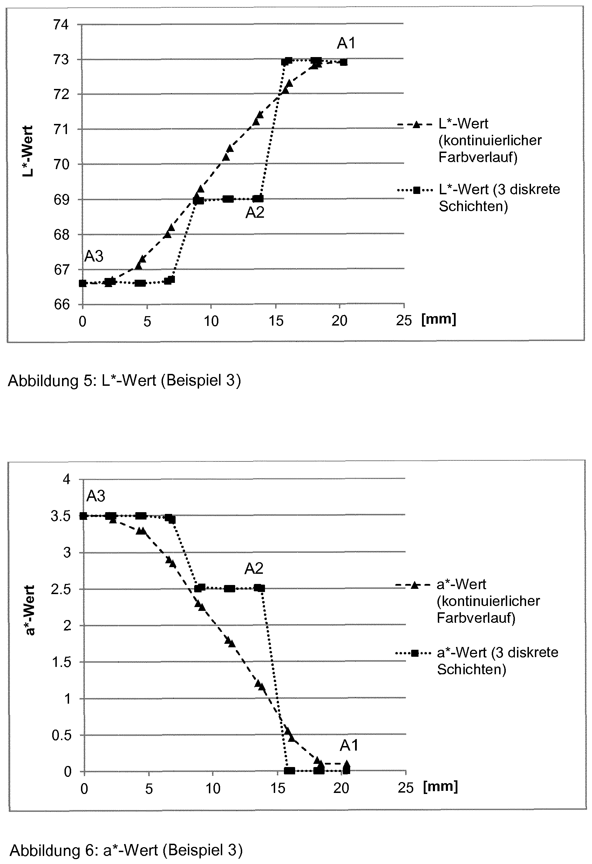

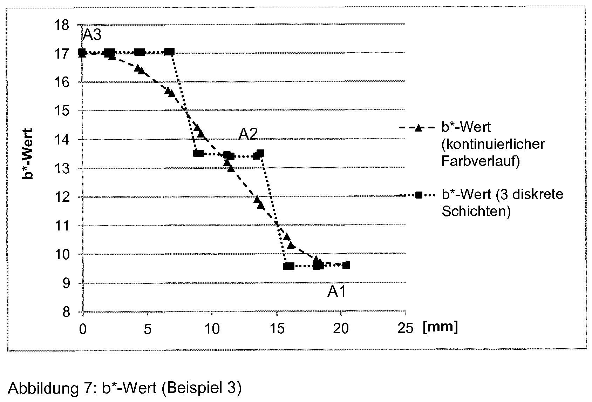

Der Vergleichsblock besteht aus drei diskreten Schichten. Die L*a*b*-Werte der einzelnen Schichten entsprechen jeweils den L*a*b*-Werten der drei einzelnen Pasten 2-A1, 2-A2 und 2-A3. Von einer zur anderen Schicht ändern sich die L*a*b*-Werte sprunghaft (vgl. Abbildungen 5 bis 7). Visuell sind die einzelnen Schichten deutlich voneinander zu unterscheiden.

Demgegenüber wird durch Dosierung von zwei unterschiedlich gefärbten Pasten mit sich ändernder Dosiergeschwindigkeit ein Block erhalten, bei dem sich die L*a*b*-Werte kontinuierlich ändern (vgl. Abbildungen 5 bis 7). Bei diesem Block sind visuell keine Farbsprünge feststellbar.

**Tabelle 5 (Biegefestigkeiten):**

| | 2-A1 | 2-A2 | 2-A3 | Block mit 3 diskreten Schichten | Block mit kontinuierlichem Farbverlauf |
|---|---|---|---|---|---|
| BBF | 201 MPa | 197 MPa | 198 MPa | 186 MPa | 200 MPa |
| 3PBF | 177 MPa | 179 MPa | 177 MPa | 175 MPa* | 179 MPa |
| E-Modul | 13.2 GPa | 13.2 GPa | 13.2 GPa | 13.1 GPa* | 13.2 GPa |

| | | | | | |
|---|---|---|---|---|---|
| * 2 von 10 Probekörpern versagten vorzeitig an der Grenzfläche zwischen zwei Farben und wurden nicht in die Berechnung der Mittelwerte miteinbezogen. | | | | | |

Die drei einfarbigen Vergleichsblöcke weisen die folgenden L*a*b*-Farbwerte auf.

**Tabelle 4 (Farbwerte einfarbiger Blöcke aus den Pasten 2-A1, 2-A2 und 2-A3):**

| | 2-A1 | 2-A2 | 2-A3 |
|---|---|---|---|
| L*-Wert | 73.0 | 69.0 | 66.6 |
| a*-Wert | 0.0 | 2.5 | 3.5 |
| b*-Wert | 9.5 | 13.5 | 17.0 |

Der Vergleichsblock besteht aus drei diskreten Schichten. Die L*a*b*-Werte der einzelnen Schichten entsprechen jeweils den L*a*b*-Werten der drei einzelnen Pasten 2-A1, 2-A2 und 2-A3. Von einer zur anderen Schicht ändern sich die L*a*b*-Werte sprunghaft (vgl. Abbildungen 5 bis 7). Visuell sind die einzelnen Schichten deutlich voneinander zu unterscheiden.

Demgegenüber wird durch Dosierung von zwei unterschiedlich gefärbten Pasten mit sich ändernder Dosiergeschwindigkeit ein Block erhalten, bei dem sich die L*a*b*-Werte kontinuierlich ändern (vgl. Abbildungen 5 bis 7). Bei diesem Block sind visuell keine Farbsprünge feststellbar.

**Tabelle 5 (Biegefestigkeiten):**

| | 2-A1 | 2-A2 | 2-A3 | Block mit 3 diskreten Schichten | Block mit kontinuierlichem Farbverlauf |
|---|---|---|---|---|---|
| BBF | 201 MPa | 197 MPa | 198 MPa | 186 MPa | 200 MPa |
| 3PBF | 177 MPa | 179 MPa | 177 MPa | 175 MPa* | 179 MPa |
| E-Modul | 13.2 GPa | 13.2 GPa | 13.2 GPa | 13.1 GPa* | 13.2 GPa |

| | | | | | |
|---|---|---|---|---|---|
| * 2 von 10 Probekörpern versagten vorzeitig an der Grenzfläche zwischen zwei Farben und wurden nicht in die Berechnung der Mittelwerte miteinbezogen. | | | | | |

## Patentansprüche

1. Fräsrohling zur Herstellung einer indirekten dentalen Restauration aus Kunststoff oder aus einem kunststoffbasierten Komposit, **dadurch gekennzeichnet, dass** sich die Farbe kontinuierlich von einer Fläche zur gegenüberliegenden Fläche ändert,
wobei sich der L*-Wert und der a*-Wert und der b*-Wert im L*a*b*-Farbraum kontinuierlich von einer Fläche zur gegenüberliegenden Fläche ändern und
wobei
die Differenz ΔL* = |L*2 - L*1| größer 2 und
die Differenz Δa* = |a*1 - a*2| größer 0.5 und
die Differenz Δb* = |b*1 - b*2| größer 1 ist,
wobei die L*a*b*-Werte an 2 mm dicken Scheiben gegen einen weißen Hintergrund gemessen werden.

2. Fräsrohling nach Anpruch 1, wobei die Differenz ΔL* = |L*2 - L*1| größer 4, und
die Differenz Δa* = |a*1 - a*2| größer 1 und
die Differenz Δb* = |b*1 - b*2| größer 2 ist,
wobei die L*a*b*-Werte an 2 mm dicken Scheiben gegen einen weißen Hintergrund gemessen werden.

3. Fräsrohling nach einem der vorangehenden Ansprüche, wobei
L*1 im Bereich von 50 bis 70, bevorzugt von 50 bis 68,
L*2 im Bereich von 65 bis 85, bevorzugt von 68 bis 85,
a*1 im Bereich von 3 bis 7, bevorzugt von 3.5 bis 7,
a*2 im Bereich von -2 bis 4, bevorzugt von -2 bis 3.5,
b*1 im Bereich von 12 bis 22, bevorzugt von 14 bis 22, und
b*2 im Bereich von 5 bis 15, bevorzugt von 5 bis 14, liegt,
wobei die L*a*b*-Werte an 2 mm dicken Scheiben gegen einen weißen Hintergrund gemessen werden.

4. Fräsrohling nach einem der vorangehenden Ansprüche, der x-, y- und z-Dimensionen von mindestens 5 mm, bevorzugt mindestens 10 mm besonders bevorzugt mindestens 14 mm aufweist.

5. Dentaler Formkörper hergestellt aus einem Fräsrohling gemäß der Ansprüche 1 bis 4.

6. Dentaler Formkörper nach Anspruch 5, wobei es sich um eine Krone, eine Teilkrone, eine Brücke, ein Inlay, ein Onlay, ein Veneer, einen teil- oder vollanatomischen künstlichen Zahn oder ein Abutment handelt.

7. Verfahren zur Herstellung eines Fräsrohlings für die Herstellung einer indirekten dentalen Restauration aus Kunststoff oder aus einem kunststoffbasierten Komposit nach Anspruch 1 bis 4, umfassend die folgenden Schritte
- Herstellen oder Bereitstellen einer ersten radikalisch härtbaren Zusammensetzung mit einer ersten Farbe,
- Herstellen oder Bereitstellen einer zweiten radikalisch härtbaren Zusammensetzung mit einer zweiten Farbe,
- Befüllen einer Form unter Mischen der ersten und der zweiten radikalisch härtbaren Zusammensetzungen und
- Aushärten der Mischung der ersten und der zweiten Zusammensetzung,
wobei das Mischungsverhältnis der ersten und der zweiten radikalisch härtbaren Zusammensetzungen beim Befüllen der Form von einer Fläche zur gegenüberliegenden Fläche kontinuierlich variiert wird.

8. Verfahren nach Anspruch 7, wobei das Mischen und Befüllen mit einer Förder- oder Pressvorrichtung, bevorzugt mit einer Extrusionsvorrichtung erfolgt.

9. Verfahren nach einem der Ansprüche 7 und 8, wobei mit einem Extrusionsdruck im Bereich von 20 bis 200 bar und/oder einer Extrusionsgeschwindigkeit im Bereich von 0.1 bis 100 mm/min, bevorzugt von 1 bis 30 mm/min, und/oder unter Anlegen eines Vakuums, bevorzugt im Bereich von -0.5 bis -1.0 bar, befüllt wird.

## Claims

1. Milling blank for production of an indirect dental restoration composed of resin or of resin-based composite, **characterized in that** the colour changes continuously from one surface to the opposite surface, wherein the L* value and the a* value and the b* value in the L*a*b* colour space change continuously from one surface to the opposite surface and wherein
the difference ΔL* = |L*2 - L*1| is greater than 2 and
the difference Δa* = |a*1 - a*2| is greater than 0.5 and
the difference Δb* = |b*1 - b*2| is greater than 1,
wherein the L*a*b* values are measured on 2 mm-thick discs against a white background.

2. Milling blank according to claim 1, wherein
the difference ΔL* = |L*2 - L*1| is greater than 4 and
the difference Δa* = |a*1 - a*2| is greater than 1 and
the difference Δb* = |b*1 - b*2| is greater than 2,
wherein the L*a*b* values are measured on 2 mm-thick discs against a white background.

3. Milling blank according to any of the preceding claims, wherein
L*1 is in the range from 50 to 70, preferably from 50 to 68,
L*2 is in the range from 65 to 85, preferably from 68 to 85,
a*1 is in the range from 3 to 7, preferably from 3.5 to 7,
a*2 is in the range from -2 to 4, preferably from -2 to 3.5,
b*1 is in the range from 12 to 22, preferably from 14 to 22, and
b*2 is in the range from 5 to 15, preferably from 5 to 14,
wherein the L*a*b* values are measured on 2 mm-thick discs against a white background.

4. Milling blank according to any of the preceding claims, having x, y, and z dimensions of at least 5 mm, preferably at least 10 mm, more preferably at least 14 mm.

5. Dental shaped body produced from a milling blank according to claims 1 to 4.

6. Dental shaped body according to claim 5, which is a crown, a partial crown, a bridge, an inlay, an onlay, a veneer, a partly or fully anatomical artificial tooth or an abutment.

7. Method of producing a milling blank for the production of an indirect dental restoration composed of resin or composed of a resin-based composite according to claims 1 to 4, comprising the following steps:
- producing or providing a first free-radically curable composition having a first colour,
- producing or providing a second free-radically curable composition having a second colour,
- filling a mould while mixing the first and second free-radically curable compositions and
- curing the mixture of the first and second compositions,
wherein the mixing ratio of the first and second free-radically curable compositions is varied continuously in the filling of the mold from one surface to the opposite surface.

8. Method according to Claim 7, wherein the mixing and filling are effected with a conveying or pressing apparatus, preferably with an extrusion apparatus.

9. Method according to any of Claims 7 and 8, wherein filling is effected with an extrusion pressure in the range from 20 to 200 bar and/or at an extrusion rate in the range from 0.1 to 100 mm/min, preferably from 1 to 30 mm/min, and/or with application of a reduced pressure, preferably in the range from -0.5 to -1.0 bar.

## Revendications

1. Ébauche de fraisage pour la fabrication d'une restauration dentaire indirecte en matériau synthétique ou en un composite à base de matériau synthétique, **caractérisée en ce que** la couleur se modifie en continu d'une surface vers la surface opposée, la valeur L* et la valeur a* et la valeur b* dans l'espace chromatique L*a*b* se modifiant en continu d'une surface vers la surface opposée et
la différence ΔL* = |L*2-L*1| étant supérieure à 2 et
la différence Δa* = |a*1-a*2| étant supérieure à 0,5 et
la différence Δb* = |b*1-b*2| étant supérieure à 1,
les valeurs L*a*b* étant mesurées sur des plaquettes de 2 mm d'épaisseur par rapport à un fond blanc.

2. Ébauche de fraisage selon la revendication 1, la différence ΔL* = |L*2-L*1| étant supérieure à 4 et
la différence Δa* = |a*1-a*2| étant supérieure à 1 et
la différence Δb* = |b*1-b*2| étant supérieure à 2,
les valeurs L*a*b* étant mesurées sur des plaquettes de 2 mm d'épaisseur par rapport à un fond blanc.

3. Ébauche de fraisage selon l'une quelconque des revendications précédentes,
L*1 étant situé dans la plage de 50 à 70, de préférence de 50 à 68,
L*2 étant situé dans la plage de 65 à 85, de préférence de 68 à 85,
a*1 étant situé dans la plage de 3 à 7, de préférence de 3,5 à 7,
a*2 étant situé dans la plage de -2 à 4, de préférence de -2 à 3,5,
b*1 étant situé dans la plage de 12 à 22, de préférence de 14 à 22 et
b*2 étant situé dans la plage de 5 à 15, de préférence de 5 à 14,
les valeurs L*a*b* étant mesurées sur des plaquettes de 2 mm d'épaisseur par rapport à un fond blanc.

4. Ébauche de fraisage selon l'une quelconque des revendications précédentes, qui présente des dimensions x, y et z d'au moins 5 mm, de préférence d'au moins 10 mm et de manière particulièrement préférée d'au moins 14 mm.

5. Corps façonné dentaire fabriqué à partir d'une ébauche de fraisage selon l'une quelconque des revendications 1 à 4.

6. Corps façonné dentaire selon la revendication 5, où il s'agit d'une couronne, d'une couronne partielle, d'un bridge, d'un inlay, d'un onlay, d'un placage, d'une dent artificielle partiellement ou complètement anatomique ou d'un pilier.

7. Procédé pour la fabrication d'une ébauche de fraisage pour la fabrication d'une restauration dentaire indirecte en matériau synthétique ou en un composite à base de matériau synthétique selon la revendication 1 à 4, comprenant les étapes suivantes
- fabrication ou utilisation d'une première composition durcissable par voie radicalaire d'une première couleur,
- fabrication ou utilisation d'une deuxième composition durcissable par voie radicalaire d'une deuxième couleur,
- remplissage d'un moule tout en mélangeant la première et la deuxième composition durcissables par voie radicalaire et
- durcissement du mélange de la première et de la deuxième composition,
le rapport de mélange de la première et de la deuxième composition durcissables par voie radicalaire étant varié en continu lors du remplissage du moule d'une surface à la surface opposée.

8. Procédé selon la revendication 7, le mélange et le remplissage ayant lieu à l'aide d'un dispositif de transport ou de pressage, de préférence à l'aide d'un dispositif d'extrusion.

9. Procédé selon l'une quelconque des revendications 7 et 8, le remplissage étant effectué à une pression d'extrusion dans la plage de 20 à 200 bars et/ou à une vitesse d'extrusion dans la plage de 0,1 à 100 mm/min, de préférence de 1 à 30 mm/min et/ou avec application d'un vide, de préférence dans la plage de -0,5 à -1,0 bar.
